# EUROPEAN PATENT APPLICATION

(11) **EP 4 541 353 A1**
(43) Date of publication of application: **23.04.2025**
(21) Application number: 23823254.0
(22) Date of filing: 16.06.2023
(51) Int. Cl.: A61K 31/4365, A61K 9/20, A61P 7/02, A61P 9/10, A61P 25/00

(54) **PHARMACEUTICAL COMPOSITION FOR TREATING AND RESISTING BLOOD COAGULATION AND USE THEREOF**

(30) Priority: 17.06.2022 CN 202210688248
(71) Applicant: Chengdu Shibeikang Biomedical Technology Co., Ltd., Chengdu, Sichuan 611730 (CN)
(72) Inventor: ZHANG, Hai, Chengdu, Sichuan 611730 (CN); NIE, Yu, Chengdu, Sichuan 611730 (CN); MOU, Xia, Chengdu, Sichuan 611730 (CN); SU, Yongwen, Chengdu, Sichuan 611730 (CN); YI, Zeqin, Chengdu, Sichuan 611730 (CN)
(74) Representative: Prinz & Partner mbB
(86) International application number: PCT/CN2023/100638
(87) International publication number: WO 2023/241688

(57) **Abstract**

The present invention relates to a composition for treating and resisting blood coagulation and use thereof, and particularly relates to a pharmaceutical composition containing (7aS, 2'S)-2-oxo-clopidogrel and use thereof. The present invention provides a medicinal specification of a solid preparation which, after oral administration before/after meals for use in healthy adults, can be similar to clopidogrel bisulfate tablets that are 12.5 times larger in specification in terms of Cₘₐₓ, AUC₍₀₋ₜ₎, and AUC_{(0-∞)} of a metabolically produced compound A and an active metabolite H4, thereby ensuring the effectiveness and safety of usage of medication.

## Description

This application claims the priority of Patent Application No. CN202210688248.6, filed on June 17, 2022, and titled with "PHARMACEUTICAL COMPOSITION FOR TREATING AND RESISTING BLOOD COAGULATION AND USE THEREOF", the disclosure of which is hereby incorporated by reference in its entirety.

### FIELD

The present invention relates to a composition for treatment of blood coagulation and use thereof, and in particular to a pharmaceutical composition comprising (7aS, 2'S)-2-oxo-clopidogrel and use thereof.

### BACKGROUND

(7aS, 2'S)-2-oxo-clopidogrel (Compound A) is a metabolite of clopidogrel in the human body, with a chemical name of methyl (S)-2-(2-chlorophenyl)-2-((S)-2-oxo-2,6,7,7a-tetrahydrothiophene[3,2-c]pyridin-5(4H)-yl)acetate

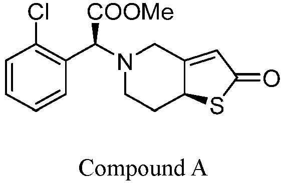

Compound A, like clopidogrel, is further activated via metabolism to the active metabolite H4 in the human body (Literature 1: Cai Liu, Yifan Zhang, Weili Chen, etc. Pharmacokinetics and pharmacokinetic/pharmacodynamic relationship of vicagrel, a novel thienopyridine P2Y12 inhibitor, compared with clopidogrel in healthy Chinese subjects following single oral dosing. Phasci (2018), doi:10.1016/j.ejps.2018.10.011), which in turn selectively inhibits binding of adenosine diphosphate (ADP) to P2Y12 receptor on platelet and the secondary ADP-mediated activation of the glycoprotein GPIIb/IIIa complex, thereby inhibiting platelet aggregation. Although clopidogrel is currently the first-line medication used clinically to prevent and treat diseases related to cardiac, cerebral and other arterial circulatory disorders caused by platelet aggregation, its efficacy, particularly among Asians, shows significant individual variability, in other words, there is a clopidogrel resistance (CPGR). Recent studies have shown that the difference in the activity of the CYP2C19 enzyme in individuals' livers is responsible for CPGR, which means that some patients fail to properly metabolize clopidogrel in they livers, leading to little or no production of the metabolite of compound A and optical isomers thereof, which blocks the subsequent metabolism of clopidogrel into its active form and prevents it from exerting anticoagulant effects. Direct oral administration of compound A can effectively avoid clopidogrel resistance. However, there have been no reports on the bioequivalence between compound A and clopidogrel to date.

Compound A is an off-white solid powder with very poor solubility, which is very slightly soluble in water, slightly soluble in ethanol, hardly soluble in methanol, almost insoluble in toluene and very slightly soluble in acetone, which belongs to a low solubility and high permeability drugs in BCS (Biopharmaceutics Classification System), and therefore these properties increase the difficulty of its bioequivalence to clopidogrel. Moreover, like clopidogrel, it has a bleeding risk and requires careful control of peak blood concentration; too low a blood drug concentration will not achieve the therapeutic effect, and too high a blood drug concentration will lead to bleeding and other fatal safety issues. In addition, a 2-step metabolism of clopidogrel in liver is required to produce the active form H4, and some people have clopidogrel resistance. However, compound A only requires one-step metabolism, so there are still very great technical difficulties to achieve consistent Tmax and the bioequivalence of Cmax and AUC of the two in the whole population.

Therefore, the study of a pharmaceutical formulation of compound A that are bioequivalent to marketed clopidogrel (Plavix^{®}, 75 mg) and can achieve a sustained, stable, and complete release of drug to ensure the efficacy and safety of the medication has become an urgent clinical challenge in this field.

### SUMMARY

In view of this, one object of the present invention is to provide a pharmaceutical composition comprising compound A for the treatment and/or prevention of a blood coagulation-related disease, and use thereof. The pharmaceutical composition comprising compound A acts by a sustained, stable and complete release of the active ingredient, and is useful in the treatment and/or prevention of cardiac, cerebral and other arterial circulatory disorders due to platelet aggregation, including, but not limited to, the treatment and/or prevention of ischemic stroke, heart attack, acute coronary syndrome, myocardial infarction, percutaneous coronary intervention, peripheral arterial disease, platelet aggregation inhibition, and transient ischemic attack.

The present disclosure provides use of compound A in the manufacture of a solid preparation for the treatment and/or prevention of a blood coagulation-related disease, wherein the solid preparation comprises a therapeutically effective amount of compound A with a particle size D90 of 3 µm to 8 µm, a single daily dose of the solid preparation provides a mean Cₘₐₓ of compound A of 0.8 ng/ml to 1.2 ng/ml, and compound A is (7aS, 2'S)-2-oxo-clopidogrel represented by:

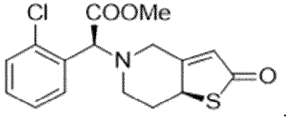

In some embodiments, the therapeutically effective amount is 4 mg to 5.5 mg in a single daily dose, and preferably, the therapeutically effective amount is 4 mg to 5 mg in a single daily dose.

In some embodiments, the single daily dose provides a mean Cₘₐₓ/C₁₂ₕ of compound A of 8 to 12.

In some embodiments, compound A has a particle size D90 of 4 µm to 6 µm.

In some embodiments, the therapeutically effective amount is 4 mg, 4.1 mg, 4.2 mg, 4.3 mg, 4.4 mg, 4.5 mg, 4.6 mg, 4.7 mg, 4.8 mg, 4.9 mg, 5 mg, 5.1 mg, 5.2 mg, 5.3 mg, 5.4 mg or 5.5mg in a single daily dose, preferably 4 mg, 4.5 mg or 5.5 mg.

In some embodiments, the solid formulation comprises a binder.

In some embodiments, the binder is selected from the group consisting of gum arabic, gelatin, sodium carboxymethylcellulose, methylcellulose, copovidone, polyvidone, hypromellose, and a combination thereof.

In some embodiments, the solid preparation for the above use further comprises a filler, a disintegrant and a lubricant as a pharmaceutically acceptable excipient.

In some embodiments, the filler includes, but not limited to, mannitol, sorbitol, calcium sulfate, calcium hydrogen phosphate, calcium carbonate, calcium sulfate dihydrate, sucrose, dextrin, microcrystalline cellulose, lactose, starch, pregelatinized starch, powdered sugar, dextrin, and a combination thereof.

In some embodiments, the disintegrant includes, but not limited to, low-substituted hydroxypropyl cellulose, polacrilin potassium, crospovidone, cross-linked sodium carboxymethyl cellulose, sodium carboxymethyl starch, dry starch, and a combination thereof.

In some embodiments, the lubricant includes, but not limited to, magnesium stearate, sodium stearic fumarate, silicon dioxide, talc powder, polyethylene glycol-like substances, hydrogenated vegetable oils, sodium dodecyl sulfate, and a combination thereof.

In some embodiments, the therapeutically effective amount is 4 mg in a single daily dose, and the single daily dose provides a mean Cₘₐₓ of compound A ranging from 80% to 125% of 0.8 ng/ml.

In some embodiments, the therapeutically effective amount is 4.5 mg in a single daily dose, and the single daily dose provides a mean Cₘₐₓ of compound A ranging from 80% to 125% of 0.9 ng/ml.

In some embodiments, the therapeutically effective amount is 5.5 mg in a single daily dose, and the single daily dose provides a mean Cₘₐₓ of compound A ranging from 80% to 125% of 1.2 ng/ml.

In some embodiments, the therapeutically effective amount is 4 mg in a single daily dose, and the single daily dose provides a mean AUC₍₀₋₄₈ₕ₎ of compound A ranging from 80% to 125% of 2.3h*ng/ml.

In some embodiments, the therapeutically effective amount is 4.5 mg in a single daily dose, and the single daily dose provides a mean AUC₍₀₋₄₈ₕ₎ of compound A ranging from 80% to 125% of 3.0h*ng/ml.

In some embodiments, the therapeutically effective amount is 5.5 mg in a single daily dose, and the single daily dose provides a mean AUC₍₀₋₄₈ₕ₎ of compound A ranging from 80% to 125% of 3.5h*ng/ml.

In some embodiments, the therapeutically effective amount is 4 mg in a single daily dose, and the single daily dose provides a mean AUC₍₀₋ₜ₎ of compound A ranging from 80% to 125% of 2.3h*ng/ml.

In some embodiments, the therapeutically effective amount is 4.5 mg in a single daily dose, and the single daily dose provides a mean AUC₍₀₋ₜ₎ of compound A ranging from 80% to 125% of 3.0h*ng/ml.

In some embodiments, the therapeutically effective amount is 5.5 mg in a single daily dose, and the single daily dose provides a mean AUC₍₀₋ₜ₎ of compound A ranging from 80% to 125% of 3.5h*ng/ml.

In some embodiments, the therapeutically effective amount is 4 mg in a single daily dose, and the single daily dose provides a mean AUC_{(0-∞)} of compound A ranging from 80% to 125% of 2.7h*ng/ml.

In some embodiments, the therapeutically effective amount is 4.5 mg in a single daily dose, and the single daily dose provides a mean AUC_{(0-∞)} of compound A ranging from 80% to 125% of 3.5h*ng/ml.

In some embodiments, the therapeutically effective amount is 5.5 mg in a single daily dose, and the single daily dose provides a mean AUC_{(0-∞)} of compound A ranging from 80% to 125% of 4.2h*ng/ml.

In some embodiments, the therapeutically effective amount is 4mg to 5.5 mg in a single daily dose, and the single daily dose provides a mean AUC₍₀₋₄₈ₕ₎ of compound A of 2.3 to 3.5 h*ng/ml following administration to a human subject.

In some embodiments, the therapeutically effective amount is 4mg to 5.5 mg in a single daily dose, and the single daily dose provides a mean AUC₍₀₋ₜ₎ of compound A of 2.3 to 3.5 h*ng/ml following administration to a human subject.

In some embodiments, the therapeutically effective amount is 4mg to 5.5 mg in a single daily dose, and the single daily dose provides a mean AUC_{(0-∞)} of compound A of 2.7 to 4.2 h*ng/ml following administration to a human subject.

In some embodiments, the therapeutically effective amount is 4mg to 5.5 mg in a single daily dose, and the single daily dose provides a mean AUC_{(0-∞)} of an active metabolite H4 of 13.5 to 19 h*ng/ml following administration to a human subject.

In some embodiments, the therapeutically effective amount is 4mg to 5.5 mg in a single daily dose, and the single daily dose provides a mean AUC₍₀₋ₜ₎ of an active metabolite H4 of 13 to 18.5 h*ng/ml following administration to a human subject.

In some embodiments, the therapeutically effective amount is 4mg to 5.5 mg in a single daily dose, and the single daily dose provides a mean AUC₍₀₋₁₂ₕ₎ of an active metabolite H4 of 13 to 18.5 h*ng/ml following administration to a human subject.

In some embodiments, the therapeutically effective amount is 4mg to 5.5 mg in a single daily dose, and the single daily dose provides a mean Cₘₐₓ of an active metabolite H4 of 12.5 to 17 ng/ml following administration to a human subject.

In some embodiments, the therapeutically effective amount is 4 mg in a single daily dose, and the single daily dose provides a mean AUC₍₀₋₁₂ₕ₎ of an active metabolite H4 ranging from 80% to 125% of 13h*ng/ml.

In some embodiments, the therapeutically effective amount is 4.5 mg in a single daily dose, and the single daily dose provides a mean AUC₍₀₋₁₂ₕ₎ of an active metabolite H4 ranging from 80% to 125% of 14h*ng/ml.

In some embodiments, the therapeutically effective amount is 5.5 mg in a single daily dose, and the single daily dose provides a mean AUC₍₀₋₁₂ₕ₎ of an active metabolite H4 ranging from 80% to 125% of 18.5h*ng/ml.

In some embodiments, the therapeutically effective amount is 4 mg in a single daily dose, and the single daily dose provides a mean AUC₍₀₋ₜ₎ of an active metabolite H4 ranging from 80% to 125% of 13h*ng/ml.

In some embodiments, the therapeutically effective amount is 4.5 mg in a single daily dose, and the single daily dose provides a mean AUC₍₀₋ₜ₎ of an active metabolite H4 ranging from 80% to 125% of 14h*ng/ml.

In some embodiments, the therapeutically effective amount is 5.5 mg in a single daily dose, and the single daily dose provides a mean AUC₍₀₋ₜ₎ of an active metabolite H4 ranging from 80% to 125% of 18.5h*ng/ml.

In some embodiments, the therapeutically effective amount is 4 mg in a single daily dose, and the single daily dose provides a mean AUC_{(0-∞)} of an active metabolite H4 ranging from 80% to 125% of 13.5h*ng/ml.

In some embodiments, the therapeutically effective amount is 4.5 mg in a single daily dose, and the single daily dose provides a mean AUC_{(0-∞)} of an active metabolite H4 ranging from 80% to 125% of 15h*ng/ml.

In some embodiments, the therapeutically effective amount is 5.5 mg in a single daily dose, and the single daily dose provides a mean AUC_{(0-∞)} of an active metabolite H4 ranging from 80% to 125% of 19h*ng/ml.

In some embodiments, the therapeutically effective amount is 4 mg in a single daily dose, and the single daily dose provides a mean Cₘₐₓ of an active metabolite H4 ranging from 80% to 125% of 12.5ng/ml.

In some embodiments, the therapeutically effective amount is 4.5 mg in a single daily dose, and the single daily dose provides a mean Cₘₐₓ of an active metabolite H4 ranging from 80% to 125% of 14ng/ml.

In some embodiments, the therapeutically effective amount is 5.5 mg in a single daily dose, and the single daily dose provides a mean Cₘₐₓ of an active metabolite H4 ranging from 80% to 125% of 17ng/ml.

The present disclosure further provides a pharmaceutical composition for use in the treatment and/or prevention of a blood coagulation-related disease, comprising a therapeutically effective amount of compound A and at least one pharmaceutically acceptable excipient, wherein compound A has a particle size D90 of 3µm to 8µm, the excipient comprises a binder, and compound A is (7aS, 2'S)- 2-oxo-clopidogrel represented by:

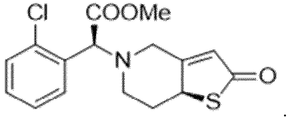

In some embodiments, the therapeutically effective amount is 4 mg to 5.5 mg in a single daily dose, and preferably, the therapeutically effective amount is 4 mg to 5 mg in a single daily dose.

In some embodiments, a single daily dose provides a mean Cₘₐₓ of compound A of 0.8ng/ml to 1.2ng/ml following administration of a therapeutically effective amount of the pharmaceutical composition to a human subject.

In some embodiments, the binder includes, but not limited to, gum arabic, gelatin, sodium carboxymethylcellulose, methylcellulose, copovidone, polyvidone, hypromellose, and a combination thereof.

In some embodiments, the pharmaceutical composition further comprises a filler, a disintegrant and a lubricant as a pharmaceutically acceptable excipient.

In some embodiments, the filler includes, but not limited to, mannitol, sorbitol, calcium sulfate, calcium hydrogen phosphate, calcium carbonate, calcium sulfate dihydrate, sucrose, dextrin, microcrystalline cellulose, lactose, starch, pregelatinized starch, powdered sugar, dextrin, and a combination thereof.

In some embodiments, the disintegrant includes, but not limited to, low-substituted hydroxypropyl cellulose, polacrilin potassium, crospovidone, cross-linked sodium carboxymethyl cellulose, sodium carboxymethyl starch, dry starch, and a combination thereof.

In some embodiments, the lubricant includes, but not limited to, magnesium stearate, sodium stearic fumarate, silicon dioxide, talc powder, polyethylene glycol-like substances, hydrogenated vegetable oils, sodium dodecyl sulfate, and a combination thereof.

In some embodiments, the pharmaceutical composition has a dissolution amount of greater than or equal to 70% within 30 minutes in an acetate buffer solution with a pH of 4.5, as determined by Method 2 of China Pharmacopoeia (2020 edition) General Principles 0931.

In some embodiments, the pharmaceutical composition has a dissolution amount of greater than or equal to 85% within 60 minutes in an acetate buffer solution with a pH of 4.5, as determined by Method 2 of China Pharmacopoeia (2020 edition) General Principles 0931.

In some embodiments, the single daily dose of 4 mg to 5.5 provides a mean AUC₍₀₋₄₈ₕ₎ of compound A of 2.3 to 3.5 h*ng/ml following administration of the pharmaceutical composition to a human subject.

In some embodiments, the single daily dose of 4 mg to 5.5 provides a mean AUC₍₀₋ₜ₎ of compound A of 2.3 to 3.5 h*ng/ml following administration of the pharmaceutical composition to a human subject.

In some embodiments, the single daily dose of 4 mg to 5.5 provides a mean AUC_{(0-∞)} of compound A of 2.7 to 4.2 h*ng/ml following administration of the pharmaceutical composition to a human subject.

In some embodiments, the single daily dose of 4 mg to 5.5 provides a Cₘₐₓ/C₂₄ₕ of compound A of 8 to 12 following administration of the pharmaceutical composition to a human subject.

In some embodiments, the therapeutically effective amount is 4 mg to 5.5 mg in a single daily dose, and the single daily dose provides a mean AUC_{(0-∞)} of an active metabolite H4 of 13.5 to 19 h*ng/ml following administration to a human subject.

In some embodiments, the therapeutically effective amount is 4 mg to 5.5 mg in a single daily dose, and the single daily dose provides a mean AUC₍₀₋ₜ₎ of an active metabolite H4 of 13 to 18.5 h*ng/ml following administration to a human subject.

In some embodiments, the therapeutically effective amount is 4 mg to 5.5 mg in a single daily dose, and the single daily dose provides a mean AUC₍₀₋₁₂ₕ₎ of an active metabolite H4 of 13 to 18.5 h*ng/ml following administration to a human subject.

In some embodiments, the therapeutically effective amount is 4 mg to 5.5 mg in a single daily dose, and the single daily dose provides a mean Cₘₐₓ of an active metabolite H4 of 12.5 to 17 ng/ml following administration to a human subject.

In some embodiments, the therapeutically effective amount is 4 mg in a single daily dose, and the single daily dose provides a mean Cₘₐₓ of compound A ranging from 80% to 125% of 0.8 ng/ml.

In some embodiments, the therapeutically effective amount is 4.5 mg in a single daily dose, and the single daily dose provides a mean Cₘₐₓ of compound A ranging from 80% to 125% of 0.9 ng/ml.

In some embodiments, the therapeutically effective amount is 5.5 mg in a single daily dose, and the single daily dose provides a mean Cₘₐₓ of compound A ranging from 80% to 125% of 1.2 ng/ml.

In some embodiments, the therapeutically effective amount is 4 mg in a single daily dose, and the single daily dose provides a mean AUC₍₀₋₄₈ₕ₎ of compound A ranging from 80% to 125% of 2.3h*ng/ml.

In some embodiments, the therapeutically effective amount is 4.5 mg in a single daily dose, and the single daily dose provides a mean AUC₍₀₋₄₈ₕ₎ of compound A ranging from 80% to 125% of 3.0h*ng/ml.

In some embodiments, the therapeutically effective amount is 5.5 mg in a single daily dose, and the single daily dose provides a mean AUC₍₀₋₄₈ₕ₎ of compound A ranging from 80% to 125% of 3.5h*ng/ml.

In some embodiments, the therapeutically effective amount is 4 mg in a single daily dose, and the single daily dose provides a mean AUC₍₀₋ₜ₎ of compound A ranging from 80% to 125% of 2.3h*ng/ml.

In some embodiments, the therapeutically effective amount is 4.5 mg in a single daily dose, and the single daily dose provides a mean AUC₍₀₋ₜ₎ of compound A ranging from 80% to 125% of 3.0h*ng/ml.

In some embodiments, the therapeutically effective amount is 5.5 mg in a single daily dose, and the single daily dose provides a mean AUC₍₀₋ₜ₎ of compound A ranging from 80% to 125% of 3.5h*ng/ml.

In some embodiments, the therapeutically effective amount is 4 mg in a single daily dose, and the single daily dose provides a mean AUC_{(0-∞)} of compound A ranging from 80% to 125% of 2.7h*ng/ml.

In some embodiments, the therapeutically effective amount is 4.5 mg in a single daily dose, and the single daily dose provides a mean AUC_{(0-∞)} of compound A ranging from 80% to 125% of 3.5h*ng/ml.

In some embodiments, the therapeutically effective amount is 5.5 mg in a single daily dose, and the single daily dose provides a mean AUC_{(0-∞)} of compound A ranging from 80% to 125% of 4.2h*ng/ml.

In some embodiments, the therapeutically effective amount is 4 mg in a single daily dose, and the single daily dose provides a mean AUC₍₀₋₁₂ₕ₎ of an active metabolite H4 ranging from 80% to 125% of 13h*ng/ml.

In some embodiments, the therapeutically effective amount is 4.5 mg in a single daily dose, and the single daily dose provides a mean AUC₍₀₋₁₂ₕ₎ of an active metabolite H4 ranging from 80% to 125% of 14h*ng/ml.

In some embodiments, the therapeutically effective amount is 5.5 mg in a single daily dose, and the single daily dose provides a mean AUC₍₀₋₁₂ₕ₎ of an active metabolite H4 ranging from 80% to 125% of 18.5h*ng/ml.

In some embodiments, the therapeutically effective amount is 4 mg in a single daily dose, and the single daily dose provides a mean AUC₍₀₋ₜ₎ of an active metabolite H4 ranging from 80% to 125% of 13h*ng/ml.

In some embodiments, the therapeutically effective amount is 4.5 mg in a single daily dose, and the single daily dose provides a mean AUC₍₀₋ₜ₎ of an active metabolite H4 ranging from 80% to 125% of 14h*ng/ml.

In some embodiments, the therapeutically effective amount is 5.5 mg in a single daily dose, and the single daily dose provides a mean AUC₍₀₋ₜ₎ of an active metabolite H4 ranging from 80% to 125% of 18.5h*ng/ml.

In some embodiments, the therapeutically effective amount is 4 mg in a single daily dose, and the single daily dose provides a mean AUC_{(0-∞)} of an active metabolite H4 ranging from 80% to 125% of 13.5h*ng/ml.

In some embodiments, the therapeutically effective amount is 4.5 mg in a single daily dose, and the single daily dose provides a mean AUC_{(0-∞)} of an active metabolite H4 ranging from 80% to 125% of 15h*ng/ml.

In some embodiments, the therapeutically effective amount is 5.5 mg in a single daily dose, and the single daily dose provides a mean AUC_{(0-∞)} of an active metabolite H4 ranging from 80% to 125% of 19h*ng/ml.

In some embodiments, the therapeutically effective amount is 4 mg in a single daily dose, and the single daily dose provides a mean Cₘₐₓ of an active metabolite H4 ranging from 80% to 125% of 12.5ng/ml.

In some embodiments, the therapeutically effective amount is 4.5 mg in a single daily dose, and the single daily dose provides a mean Cₘₐₓ of an active metabolite H4 ranging from 80% to 125% of 14ng/ml.

In some embodiments, the therapeutically effective amount is 5.5 mg in a single daily dose, and the single daily dose provides a mean Cₘₐₓ of an active metabolite H4 ranging from 80% to 125% of 17ng/ml.

Although compound A is the metabolite of clopidogrel, it is different from clopidogrel in terms of chemical structure and material basis, and the active pharmaceutical ingredient in the preparation for the two is different, resulting in the dosage of compound A and clopidogrel bisulfate are also different. A 2-step metabolism of clopidogrel in liver is required to produce the active form H4, and some people have clopidogrel resistance. However, compound A only requires one-step metabolism, so there are still very great technical difficulties to achieve consistent Tₘₐₓ and the bioequivalence of Cₘₐₓ and AUC of the two in the whole population.

According to the metabolic pathway of clopidogrel (as cited Literature 1 in the background part), it has been theoretically calculated that approximately 11.25 mg of compound A is equivalent to 75 mg of clopidogrel bisulfate (15% of clopidogrel is metabolized to compound A after oral intake). However, clinical trials have revealed that it is challenging to achieve simultaneously consistent Tₘₐₓ while maintaining equivalence in Cₘₐₓ, AUC₍₀₋ₜ₎, and AUC_{(0-∞)} for the compound at that dose. Particularly, compound A, which serves as a metabolic intermediate, possesses an advantage in terms of in vivo metabolism. To achieve simultaneous consistent Tₘₐₓ, it faces an increased challenge. After extensive research on formulations and strengths with different particle sizes, the inventors surprisingly discovered that 4 mg, 5 mg and 5.5 mg of compound A with a particle size D90 controlled at 3 µm to 8 µm are potentially bioequivalent to clopidogrel bisulfate (Polivir^{®}, 75 mg) in the human body. This dose range has a difference of more than 10 folds compared to clopidogrel bisulfate (Polivir^{®}, 75 mg), which is not known in the prior art. However, solid preparations of compound A with different strengths exhibit differences in Cₘₐₓ, AUC₍₀₋ₜ₎ and AUC_{(0-∞)} of the active metabolite H4 produced therefrom in the human body, making it difficult to achieve bioequivalence, in particular due to a low Cₘₐₓ of H4. Although reducing particle size can adjust solubility, leading to an up-regulation in Cₘₐₓ and a reduction in Tₘₐₓ, simultaneously achieving bioequivalence in AUC_{(0-∞)} remains challenging. Moreover, controlling the Cₘₐₓ/C₁₂ₕ of compound A to achieve stable release effect increases the difficulty.

The inventors also found that the formulation composition has a large influence on dissolution. For example, when adding microcrystalline cellulose as a filler, although microcrystalline cellulose improves the fluidity of the particles, the dissolution rate of compound A is decreased after tableting. As another example, different amounts of disintegrants added have little influence on the dissolution rate of compound A, and different types and amounts of solubilizers have very limited influence. In addition, micronisation of drug material accelerates the dissolution of compound A, but makes the particle size too small, and therefore it can easily lead to a burst release (too rapid absorption of the drug, increasing the risk of bleeding). Therefore, conventional methods addressing the dissolution problems of poorly soluble drugs are insufficient to control the solid preparation of compound A to achieve the goal of sustained, stable, and complete release. In addition, the inventors further found during the research process that food greatly affects the absorption of compound A, primarily manifested difficulty to achieve Cₘₐₓ equivalence pre- and post-meal, which undoubtedly poses a great challenge in this field. After a large number of formulation and strength studies, the inventors surprisingly found that the addition of a binder to the solid formulation could potentially achieve complete bioequivalence to clopidogrel bisulfate (Plavix^{®}, 75 mg) both before and after meals, which makes it possible to achieve the complete bioequivalence of the solid preparation with a stable and complete release.

In summary, for the solid preparation of compound A of the present invention, it is necessary to adjust the three-dimensional dynamic equilibrium between the strength, particle size of compound A, and formulation composition, in order to achieve bioequivalence to clopidogrel bisulfate (Plavix^{®}, 75mg) both before and after meals.

The "h" in the terms such as AUC₍₀₋₁₂ₕ₎, AUC₍₀₋₄₈ₕ₎, C₁₂ₕ and the like in the present disclosure is the abbreviation for "hour" in English, a unit of time commonly used in the art.

### Beneficial Effect

The present disclosure provides a safe and effective solid preparation of compound A for use in the treatment and/or prevention of a blood coagulation-related disease for the first time. The solid preparation achieves the complete bioequivalence to the marketed clopidogrel bisulfate (Plavix^{®}, 75 mg), and in particular the complete bioequivalence both before and after meals. Furthermore, the dosage of the active ingredient compound A is less than 1/15 (one fifteenth) of that of the marketed clopidogrel bisulfate (Plavix^{®}, 75mg), and compound A in this solid preparation has a Cₘₐₓ/C₁₂ₕ of not more than 12, and Tₘₐₓ of not more than 1 h. Therefore, the solid preparation offers a rapid, stable and complete drug release, and has a good clinical performance. In addition, the solid preparation of compound A provided by the present invention has a simple preparation process, and low production cost, making it suitable for industrial production.

### DETAILED DESCRIPTION

The embodiments of the present disclosure will be described below in connection with examples. It will be understood by those skilled in the art that the following examples are intended to illustrate the present disclosure only and should not be considered as limiting the scope of the invention. Where specific techniques or conditions are not indicated in the examples, they are carried out in accordance with the techniques or conditions described in the literature in the field or in accordance with the product specification. The reagents or instruments used without indicating the manufacturer were all conventional products that were purchased commercially.

### Example I: Screening of dosage and particle size

1. Test Formulations: The formula of examples 1 to 10 of the test formulations and preparation method thereof are listed below.

**Table 1-1 Formula detail of test formulations**

| formula | compound A | particle size D90 | mannitol (mg) | pregelatinized starch (mg) | low-substituted hydroxypropyl cellulose (mg) | hydroxypropyl methylcellulose, (mg) | hydrogenated castor oil (mg) |
|---|---|---|---|---|---|---|---|
| example 1 | 4mg | 5µm | 60.9 | 60.9 | 11.2 | 5.6 | 1.4 |
| example 2 | 4.5mg | 5µm | 60.9 | 60.9 | 11.2 | 5.6 | 1.4 |
| example 3 | 5mg | 5µm | 60.9 | 60.9 | 11.2 | 5.6 | 1.4 |
| example 4 | 8mg | 5µm | 60.9 | 60.9 | 11.2 | 5.6 | 1.4 |
| example 5 | 5.5mg | 2µm | 60.9 | 60.9 | 11.2 | 5.6 | 1.4 |
| example 6 | 5.5mg | 8µm | 60.9 | 60.9 | 11.2 | 5.6 | 1.4 |
| example 7 | 4mg | 3µm | 60.9 | 60.9 | 11.2 | 5.6 | 1.4 |
| example 8 | 5.5mg | 10µm | 60.9 | 60.9 | 11.2 | 5.6 | 1.4 |
| example 9 | 4.5mg | 6µm | 60.9 | 60.9 | 11.2 | N/A | 1.4 |
| example 10 | 3mg | 5µm | 60.9 | 60.9 | 11.2 | 5.6 | 1.4 |

### Preparation method

(1) The materials were weighed according to the formula. The materials (except for hydrogenated castor oil) were added to a mixer, then compound A was added, and mixed at a speed of 10 rpm for 10 min for later use.
(2) The prescribed amount of hydrogenated castor oil was added and mixed at a speed of 10 rpm for 2 minutes for later use.
(3) The above mixture was pressed by a round shallow concave punch into tablets with a hardness of 30-50N.

### 2. Administration regimen: Administration for healthy adults on an empty stomach

Using a single-center, single-dose, random, open design, 66 enrolled healthy volunteers (women accounted for at least 1/3) were randomly divided into 11 groups, each group of 6 volunteers. Volunteers were randomized to receive either a single dose of the examples or the reference formulation (Plavix^{®}, 75 mg) orally on an empty stomach. PK (pharmacokinetic) parameters of compound A in plasma were determined.

### 3. Test results of compound A

**Table 1-2 PK parameters of compound A in test formulations in vivo**

| test formulation | Cₘₐₓ (ng/ml) | Tₘₐₓ (h) | AUC₍₀₋ₜ₎ (h*ng/ml) | AUC_{(0-∞)} (h*ng/ml) | Cₘₐₓ/C₁₂ₕ |
|---|---|---|---|---|---|
| Plavix^{®}, 75 mg | 0.952±0.517 | 0.774±0.391 | 2.835±1.004 | 3.327±1.128 | 28 |
| example 1 | 0.772±0.436 | 0.736±0.227 | 2.313±1.222 | 2.672±1.004 | 8 |
| example 2 | 0.943±0.613 | 0.791±0.382 | 2.962±1.343 | 3.473±1.332 | 9 |
| example 3 | 1.028±0.712 | 0.752±0.452 | 3.363±1.008 | 4.125±1.569 | 10 |
| example 4 | 1.638±0.389 | 0.733±0.226 | 4.016±1.517 | 4.522±2.112 | 16 |
| example 5 | 2.102±0.527 | 0.461±0.373 | 4.558±2.204 | 5.036±2.043 | 18 |
| example 6 | 1.166±0.589 | 0.921±0.474 | 3.542±1.132 | 4.155±1.022 | 12 |
| example 7 | 0.864±0.336 | 0.722±0.297 | 2.683±1.011 | 3.148±1.086 | 9 |
| example 8 | 0.787±0.416 | 1.226±0.336 | 2.148±1.558 | 2.557±1.162 | 10 |
| example 9 | 0.915±0.663 | 0.676±0.443 | 2.741±1.274 | 3.294±1.447 | 10 |
| example 10 | 0.662±0.366 | 0.716±0.217 | 2.383±1.222 | 2.368±1.132 | 8 |

| | | | | | |
|---|---|---|---|---|---|
| Note: t in AUC₍₀₋ₜ₎ in the table represents 48h. | | | | | |

As shown in the above table, it was found by screening of dosage and particle size that when the dosage was too high (example 4) or too low (example 10), the Cₘₐₓ of compound A failed to reach 80% to 125% of that the reference formulation (Plavix^{®}, 75 mg), resulting in a significant difference in bioavailability and inability to achieve bioequivalence. When the particle size was too low (example 5), the Cₘₐₓ of compound A was too high, its Tₘₐₓ was too low, the AUC₍₀₋ₜ₎ and AUC_{(0-∞)} values were all too high, and these values were all higher than 125% of those of the reference preparation. When the particle size was too high (example 8), the Tₘₐₓ was too high, the onset of action was slow, and the AUC values were all below 80% of that of the reference preparation, indicating low bioavailability and failure to achieve bioequivalence. However, when the dosage was 4 mg to 5.5 mg and the particle size D90 was controlled at 3 µm to 8 µm (examples 1 to 3, 6 to 7, and 9), Cₘₐₓ, Tₘₐₓ, AUC₍₀₋ₜ₎ and AUC_{(0-∞)} of compound A were all within the range of 80% to 125% of the corresponding parameter of the reference formulation, indicating a comparable bioavailability and predictable bioequivalence. In addition, the inventors further found that when there was no binder in the formula (example 9), the PK parameters of compound A still showed its comparable bioavailability to the reference formulation. However, the Tₘₐₓ of compound A was slightly lower, as compared to the other examples using a binder, suggesting that the release rate of the compound may be affected by the binder.

### 4. Test results of active metabolite H4

**Table 1-3 PK parameters of active metabolite H4 in vivo for each test formulation**

| test formulation | Cₘₐₓ (ng/ml) | Tₘₐₓ (h) | AUC₍₀₋ₜ₎ (h*ng/ml) | AUC_{(0-∞)} (h*ng/ml) |
|---|---|---|---|---|
| Plavix^{®}, 75 mg | 13.786±5.772 | 0.628±0.349 | 15.297±4.901 | 15.448±4.953 |
| example 1 | 12.535±4.556 | 0.663±0.452 | 12.973±5.384 | 13.536±5.327 |
| example 2 | 14.427±5.961 | 0.643±0.463 | 14.276±5.050 | 14.956±5.123 |
| example 3 | 16.735±4.339 | 0.624±0.503 | 15.864±5.563 | 16.033±4.826 |
| example 4 | 25.648±6.443 | 0.733±0.615 | 25.379±7.448 | 25.674±8.006 |
| example 5 | 17.783±5.886 | 0.468±0.323 | 17.448±6.445 | 17.668±5.994 |
| example 6 | 16.956±4.378 | 0.782±0.441 | 18.457±5.118 | 19.004±4.555 |
| example 7 | 14.163±4.025 | 0.559±0.316 | 13.885±4.504 | 14.006±4.225 |
| example 8 | 10.435±3.267 | 0.963±0.446 | 16.035±6.562 | 16.227±6.774 |
| example 9 | 14.056±4.213 | 0.514±0.552 | 13.884±4.445 | 14.163±4.385 |
| example 10 | 9.435±3.657 | 0.663±0.446 | 10.884±4.145 | 11.084±5.155 |

| | | | | |
|---|---|---|---|---|
| Note: t in the table represents 12 h. | | | | |

As shown in the above table, it was found by screening of dosage and particle size that when the dosage was too high (example 4) or too low (example 10), the Cₘₐₓ of the active metabolite H4 far exceeded 80% to 125% range of that of the reference formulation (Plavix^{®}, 75 mg), resulting in a significant difference in bioavailability and inability to achieve bioequivalence. When the particle size was too high (example 8), the Cₘₐₓ of the active metabolite H4 was much lower than 80% of that of the reference preparation, and when the particle size was too low (example 5), there was a risk of high Cₘₐₓ, both of which failed to achieve a bioavailability comparable to 80% to 125% of those of the reference preparation and achieve bioequivalence. However, when the dosage was 4 mg to 5.5 mg and the particle size D90 was controlled at 3 µm to 8 µm, Cₘₐₓ, Tₘₐₓ, AUC₍₀₋ₜ₎ and AUC_{(0-∞)} of the active metabolite H4 can be well controlled to fall into the range of 80% to 125% of those of the corresponding parameters of the reference formulation, indicating a comparable bioavailability and predictable bioequivalence. In addition, the inventors further found that when there was no binder in the formula (example 9), each measure of the active metabolite H4 still showed its comparable bioavailability to the reference formulation. However, the Tₘₐₓ of the active metabolite H4 was slightly lower, as compared to the other examples using a binder, indicating a faster drug action of onset, and suggesting that its release rate may also be affected by the binder.

### Example II Bioequivalence evaluation under the fasting condition (healthy adults)

1. Test formulations : examples 2, 6, 7 and 9 in Example I
2. Trial Protocol: A single-center, oral under fasting, random, open, two-formulation, four-period crossover design was adopted for each example, with a washout period of 7 days between period. 24 healthy subjects per test example (at least 1/3 females) were randomly divided into two groups of 12 subjects each. Subjects were randomized to receive the test formulation or the reference formulation (Plavix^{®}, 75 mg) orally on an empty stomach. Compound A and the thiol active metabolite H4 from clopidogrel in plasma were detected to evaluate the bioequivalence of the examples to the reference formulation (Plavix^{®}, 75 mg).
3. Test results

**Table 2 Results of bioequivalence evaluation under the fasting condition**

| test formulation | detection indicator | Cₘₐₓ | | AUC₍₀₋ₜ₎ | | AUC_{(0-∞)} | |
|---|---|---|---|---|---|---|---|
| | | GMR% | 90%CI | GMR% | 90%CI | GMR% | 90%CI |
| example 7 | compound A | 85.63 | 85.0-118.2 | 90.45 | 93.4-105.4 | 90.63 | 94.2-104.5 |
| | H4 | 86.22 | 88.1-106.4 | 91.52 | 91.1-106.7 | 91.66 | 92.2-107.5 |
| example 2 | compound A | 100.66 | 95.4-112.4 | 100.45 | 95.3-105.6 | 100.54 | 95.7-106.2 |
| | H4 | 98.55 | 93.1-115.6 | 100.51 | 94.6-104.9 | 100.59 | 94.2-104.2 |
| example 6 | compound A | 113.44 | 106.5-118.3 | 110.42 | 108.3-115.2 | 110.54 | 110.1-119.4 |
| | H4 | 115.88 | 104.3-118.4 | 109.45 | 107.7-118.3 | 109.83 | 108.3-117.4 |
| example 9 | compound A | 95.45 | 93.4-100.8 | 100.42 | 90.4-102.5 | 100.52 | 101.5-108.4 |
| | H4 | 97.33 | 96.2-102.3 | 100.32 | 91.3-100.9 | 100.46 | 98.4-106.3 |

| | | | | | | | |
|---|---|---|---|---|---|---|---|
| Note: t in the table represents 48 h for compound A and 12 h for the active metabolite H4. | | | | | | | |

As can be seen, the results of bioequivalence evaluation prior to meal (fasting) showed that when the prescribed formula satisfies a dosage of 4 mg to 5.5 mg and a particle size of D90 of 3 µm to 8 µm, the geometric mean ratios (test formulation/reference formulation (Plavix^{®}, 75 mg)) for Cₘₐₓ, AUC₍₀₋ₜ₎ and AUC_{(0-∞)} of compound A and the active metabolite H4 were between 80% to 125%, and their 90% confidence intervals were also between 80% to 125%, indicating that the test formulations were bioequivalent to the reference formulation under a fasting condition, regardless of the presence or absence of a binder in the formula.

### Example III Bioequivalence evaluation under post-prandial condition

1. Test formulations: examples 2, 6, 7 and 9
2. Test protocol: Same as Example II, except that the oral administration was carried out after a meal.
3. Test results

**Table 3 Results of bioequivalence evaluation under post-prandial condition**

| test formulation | detection indicator | Cₘₐₓ | | AUC₍₀₋ₜ₎ | | AUC_{(0-∞)} | |
|---|---|---|---|---|---|---|---|
| | | GMR% | 90%CI | GMR% | 90%CI | GMR% | 90%CI |
| example 7 | 90.22 | 89.5-108.7 | 92.05 | 94.3-104.5 | 93.32 | 95.2-103.5 | 90.22 |
| | 89.46 | 92.1-116.4 | 94.25 | 92.3-107.6 | 96.16 | 95.5-105.7 | 89.46 |
| example 2 | 102.21 | 92.4-120.4 | 99.54 | 93.5-106.5 | 100.14 | 92.7-102.2 | 102.21 |
| | 99.85 | 95.1-115.1 | 100.16 | 96.4-109.4 | 100.29 | 91.2-102.4 | 99.85 |
| example 6 | 115.64 | 107.1-116.3 | 111.24 | 105.8-116.4 | 115.45 | 111.1-121.3 | 115.64 |
| | 114.38 | 105.1-119.4 | 109.21 | 108.2-119.1 | 109.38 | 109.3-119.2 | 114.38 |
| example 9 | 72.44 | 73.6-100.2 | 95.84 | 90.5-103.8 | 95.93 | 93.6-107.5 | 72.44 |
| | 68.42 | 58.4-100.6 | 97.33 | 89.3-104.6 | 97.42 | 94.2-108.7 | 68.42 |

| | | | | | | | |
|---|---|---|---|---|---|---|---|
| Note: t in the table represents 48 h for compound A and 12 h for the active metabolite H4. | | | | | | | |

It was found from a bioequivalence evaluation on the post-prandial condition that when the formula did not contain a binder (example 9), post-prandial bioequivalence could not be achieved, primarily due to the non-equivalence of the Cₘₐₓ of compound A and the active metabolite H4. However, when the formula satisfies a dosage of 4 mg to 5.5 mg, a particle size of D90 of 3 µm to 8 µm, and contains a binder (examples 2, 6 and 7), both the geometric mean ratios (test formulation/reference formulation (Plavix^{®}, 75 mg)) for Cₘₐₓ, AUC₍₀₋ₜ₎ and AUC_{(0-∞)} of compound A and the active metabolite H4, and their 90% confidence intervals were between 80% to 125%, indicating a bioequivalence under the post-prandial condition. This finding was somewhat fortuitous and addressed the adverse effects of food on this product, providing a more scientific option for clinical use. Additionally, throughout the development process of this product, although post-prandial bioequivalence is easily to be affected, when post-prandial equivalence is achieved (e.g., by using a binder), it can be expected that pre-prandial (fasting) bioequivalence will also be achieved.

### Example IV: Studies on different binders

1. Test formulations were formulations in examples 11 and 12. The binder in example 11 was gum arabic (3.0 mg), in example 12 was polyvidone (4.1 mg), and other components were the same as in example 2.
2. Post-prandial bioequivalence: the test method was the same as Example III.
3. Test results

**Table 4 Results of bioequivalence evaluation under post-prandial condition**

| test formulation | detection indicator | Cₘₐₓ | | AUC₍₀₋ₜ₎ | | AUC_{(0-∞)} | |
|---|---|---|---|---|---|---|---|
| | | GMR% | 90%CI | GMR% | 90%CI | GMR% | GMR% |
| example 11 | compound A | 102.52 | 94.2-106.3 | 97.44 | 96.3-108.1 | 97.54 | 95.5-105.4 |
| | H4 | 100.44 | 96.6-104.3 | 99.52 | 92.9-104.9 | 99.63 | 96.4-108.3 |
| example 12 | compound A | 108.31 | 103.3-110.8 | 104.41 | 101.6-110.3 | 104.82 | 102.2-111.5 |
| | H4 | 106.92 | 102.4-109.4 | 105.63 | 103.3-109.9 | 105.74 | 103.6-110.8 |

| | | | | | | | |
|---|---|---|---|---|---|---|---|
| Note: t in the table represents 48 h for compound A and 12 h for the active metabolite H4. | | | | | | | |

It was found from studies on binders that the formulas containing gum arabic or polyvidone as a binder could also achieve the bioequivalence, same as the formulas containing hydroxypropyl methylcellulose as a binder, suggesting that the formula, based on the dosage and particle size range disclosed herein, is not affected by the type of binder.

### Example V: Studies on post-prandial bioequivalence of capsules

1. Test formulations were formulations in examples 13 to 15, formulas and preparation methods were described below.

**Table 5-1 formulas of capsules**

| test formulation | formulation example 13 | formulation example 14 | formulation example 15 |
|---|---|---|---|
| compound A | 4 mg | 5 mg | 5.5 mg |
| particle size D90 | 8 µm | 6 µm | 3 µm |
| mannitol (mg) | 40.45 | 50.44 | 60.9 |
| pregelatinized starch (mg) | 40.45 | 50.44 | 60.9 |
| low-substituted hydroxypropyl cellulose (mg) | 7.5 | 9.38 | 11.2 |
| hydroxypropyl methylcellulose (mg) | 3.7 | 4.62 | 5.6 |
| hydrogenated castor oil (mg) | 0.9 | 1.12 | 1.41 |

Capsule preparation method is as follows.
(1) The materials were weighed according to the formula amount, each material (except for hydrogenated castor oil) was added to a mixer, then compound A was added, and mixed at a speed of 10 rpm for 10 min for later use.
(2) The prescribed amount of hydrogenated castor oil was added and mixed at a speed of 10 rpm for 2 min for later use.
(3) Capsule Filling: The above mixture was filled into sized-3 hollow capsules to obtain the capsules.

### 2. Bioequivalence evaluation method was the same as Example III.

### 3. Test results

**Table 5-2 Results of post-prandial bioequivalence of capsules**

| test formulation | detection indicator | Cₘₐₓ | | AUC₍₀₋ₜ₎ | | AUC_{(0-∞)} | |
|---|---|---|---|---|---|---|---|
| | | GMR% | 90%CI | GMR% | 90%CI | GMR% | GMR% |
| example 13 | compound A | 83.47 | 86.0-108.6 | 85.43 | 94.5-103.1 | 86.13 | 90.5-101.7 |
| | H4 | 82.68 | 86.1-104.9 | 88.37 | 90.1-105.3 | 89.07 | 91.6-105.5 |
| example 14 | compound A | 105.33 | 100.1-115.2 | 100.48 | 96.6-109.1 | 101.18 | 97.7-111.2 |
| | H4 | 103.59 | 98.1-116.8 | 102.33 | 92.2-104.1 | 102.76 | 94.2-106.1 |
| example 15 | compound A | 115.44 | 99.5-116.1 | 110.44 | 112.0-122.1 | 111.04 | 109.1-120.6 |
| | H4 | 113.94 | 101.8-122.4 | 109.18 | 103.3-116.7 | 110.81 | 109.2-118.8 |

| | | | | | | | |
|---|---|---|---|---|---|---|---|
| Note: t in the table represents 48 h for compound A and 12 h for the active metabolite H4. | | | | | | | |

It was found from studies on bioequivalence of capsules with different strengths that when the formula of capsules satisfies a dosage of 4 mg to 5.5 mg and a particle size of D90 of 3 µm to 8 µm (examples 13, 14 and 15), both the geometric mean ratios (test formulation/reference formulation (Plavix^{®}, 75 mg)) for Cₘₐₓ, AUC₍₀₋ₜ₎ and AUC_{(0-∞)} of compound A and the active metabolite H4, and their 90% confidence intervals were between 80% to 125%, indicating a bioequivalence under the post-prandial condition.

### Example VI: Dissolution assay

1. Test samples: Examples 2 and 14
2. Dissolution Assay: The sample was taken and measured for dissolution rate according to the dissolution and drug release test (China Pharmacopoeia (2020 edition) Part Four General Principle 0931 Method 2). 900 ml of hydrochloric acid solution (pH 1.2) or acetate buffer solution (pH 4.5) was used as the dissolution medium, and the rotation speed was 50 rpm (revolutions per minute). According to the method, a portion of the solution was taken and filtered at 5, 10, 15, 30, 45, 60, 90 and 120 minutes. An appropriate volume of the filtrate was measured and the absorbance was determined at 220 nm using the ultraviolet-visible spectrophotometry (General Principle 0401) to calculate the dissolution rate for each tablet.
3. Results of dissolution assay

**Table 6 Results of dissolution assay**

| sample | formulation example 2 | dissolution rate (%) | | | | | | | |
|---|---|---|---|---|---|---|---|---|---|
| | | 5 | 10 | 15 | 30 | 45 | 60 | 90 | 120 |
| | pH 1.2 | 62 | 100 | 99 | 99 | 99 | N/A | N/A | N/A |
| example 2 | pH 4.5 | 18 | 40 | 54 | 72 | 80 | 86 | 92 | 95 |
| example 14 | pH 1.2 | 52 | 99 | 100 | 99 | 99 | N/A | N/A | N/A |
| | pH 4.5 | 14 | 35 | 44 | 70 | 78 | 85 | 93 | 96 |

The examples 2 and 14 of the present disclosure exhibited a dissolution rate of greater than or equal to 70% within 30 minutes and a dissolution rate of greater than or equal to 85% within 60 minutes in an acetic acid buffer solution of pH 4.5, indicating a complete release, and a dissolution rate of greater than or equal to 99% within 10 minutes, indicating a rapidly complete release.

The above description is only the preferred embodiments of the present disclosure. It should be noted that for those skilled in the art, various modifications can be made to these examples without departing from the principle of the present disclosure, and these modifications should fall within the scope of protection of the present disclosure.

## Claims

1. Use of compound A in the manufacture of a solid preparation for the treatment and/or prevention of a blood coagulation-related disease, wherein the solid preparation comprises a therapeutically effective amount of compound A with a particle size D90 of 3 µm to 8 µm, a single daily dose of the solid preparation provides a mean Cₘₐₓ of 0.8 ng/ml to 1.2 ng/ml of compound A, and compound A is (7aS, 2'S)-2-oxo-clopidogrel represented by:

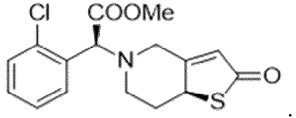

2. The use according to claim 1, wherein the therapeutically effective amount is 4 mg to 5.5 mg in a single daily dose, and preferably, the therapeutically effective amount is 4 mg to 5 mg in a single daily dose.

3. The use according to any one of claims 1 to 2, wherein the single daily dose provides a mean Cₘₐₓ/C₁₂ₕ of 8 to 12 of compound A.

4. The use according to any one of claims 1 to 2, wherein the solid formulation comprises a binder.

5. The use according to any one of claims 1 to 2, wherein the therapeutically effective amount is 4 mg in a single daily dose, and the single daily dose provides a mean Cₘₐₓ of compound A ranging from 80% to 125% of 0.8 ng/ml.

6. The use according to any one of claims 1 to 2, wherein the therapeutically effective amount is 4.5 mg in a single daily dose, and the single daily dose provides a mean Cₘₐₓ of compound A ranging from 80% to 125% of 0.9 ng/ml.

7. The use according to any one of claims 1 to 2, wherein the therapeutically effective amount is 5.5 mg in a single daily dose, and the single daily dose provides a mean Cₘₐₓ of compound A ranging from 80% to 125% of 1.2 ng/ml.

8. The use according to any one of claims 1 to 2, wherein the therapeutically effective amount is 4 mg in a single daily dose, and the single daily dose provides a mean AUC₍₀₋₄₈ₕ₎ of compound A ranging from 80% to 125% of 2.3h*ng/ml.

9. The use according to any one of claims 1 to 2, wherein the therapeutically effective amount is 4.5 mg in a single daily dose, and the single daily dose provides a mean AUC₍₀₋₄₈ₕ₎ of compound A ranging from 80% to 125% of 3.0h*ng/ml.

10. The use according to any one of claims 1 to 2, wherein the therapeutically effective amount is 5.5 mg in a single daily dose, and the single daily dose provides a mean AUC₍₀₋₄₈ₕ₎ of compound A ranging from 80% to 125% of 3.5h*ng/ml.

11. The use according to any one of claims 1 to 2, wherein the therapeutically effective amount is 4 mg in a single daily dose, and the single daily dose provides a mean AUC₍₀₋ₜ₎ of compound A ranging from 80% to 125% of 2.3h*ng/ml.

12. The use according to any one of claims 1 to 2, wherein the therapeutically effective amount is 4.5 mg in a single daily dose, and the single daily dose provides a mean AUC₍₀₋ₜ₎ of compound A ranging from 80% to 125% of 3.0h*ng/ml.

13. The use according to any one of claims 1 to 2, wherein the therapeutically effective amount is 5.5 mg in a single daily dose, and the single daily dose provides a mean AUC₍₀₋ₜ₎ of compound A ranging from 80% to 125% of 3.5h*ng/ml.

14. The use according to any one of claims 1 to 2, wherein the therapeutically effective amount is 4 mg in a single daily dose, and the single daily dose provides a mean AUC_{(0-∞)} of compound A ranging from 80% to 125% of 2.7h*ng/ml.

15. The use according to any one of claims 1 to 2, wherein the therapeutically effective amount is 4.5 mg in a single daily dose, and the single daily dose provides a mean AUC_{(0-∞)} of compound A ranging from 80% to 125% of 3.5h*ng/ml.

16. The use according to any one of claims 1 to 2, wherein the therapeutically effective amount is 5.5 mg in a single daily dose, and the single daily dose provides a mean AUC_{(0-∞)} of compound A ranging from 80% to 125% of 4.2h*ng/ml.

17. The use according to any one of claims 1 to 2, wherein the therapeutically effective amount is 4 mg in a single daily dose, and the single daily dose provides a mean AUC₍₀₋₁₂ₕ₎ of an active metabolite H4 ranging from 80% to 125% of 13h*ng/ml.

18. The use according to any one of claims 1 to 2, wherein the therapeutically effective amount is 4.5 mg in a single daily dose, and the single daily dose provides a mean AUC₍₀₋₁₂ₕ₎ of an active metabolite H4 ranging from 80% to 125% of 14h*ng/ml.

19. The use according to any one of claims 1 to 2, wherein the therapeutically effective amount is 5.5 mg in a single daily dose, and the single daily dose provides a mean AUC₍₀₋₁₂ₕ₎ of an active metabolite H4 ranging from 80% to 125% of 18.5h*ng/ml.

20. The use according to any one of claims 1 to 2, wherein the therapeutically effective amount is 4 mg in a single daily dose, and the single daily dose provides a mean AUC₍₀₋ₜ₎ of an active metabolite H4 ranging from 80% to 125% of 13h*ng/ml.

21. The use according to any one of claims 1 to 2, wherein the therapeutically effective amount is 4.5 mg in a single daily dose, and the single daily dose provides a mean AUC₍₀₋ₜ₎ of an active metabolite H4 ranging from 80% to 125% of 14h*ng/ml.

22. The use according to any one of claims 1 to 2, wherein the therapeutically effective amount is 5.5 mg in a single daily dose, and the single daily dose provides a mean AUC₍₀₋ₜ₎ of an active metabolite H4 ranging from 80% to 125% of 18.5h*ng/ml.

23. The use according to any one of claims 1 to 2, wherein the therapeutically effective amount is 4 mg in a single daily dose, and the single daily dose provides a mean AUC_{(0-∞)} of an active metabolite H4 ranging from 80% to 125% of 13.5h*ng/ml.

24. The use according to any one of claims 1 to 2, wherein the therapeutically effective amount is 4.5 mg in a single daily dose, and the single daily dose provides a mean AUC_{(0-∞)} of an active metabolite H4 ranging from 80% to 125% of 15h*ng/ml.

25. The use according to any one of claims 1 to 2, wherein the therapeutically effective amount is 5.5 mg in a single daily dose, and the single daily dose provides a mean AUC_{(0-∞)} of an active metabolite H4 ranging from 80% to 125% of 19h*ng/ml.

26. The use according to any one of claims 1 to 2, wherein the therapeutically effective amount is 4 mg in a single daily dose, and the single daily dose provides a mean Cₘₐₓ of an active metabolite H4 ranging from 80% to 125% of 12.5ng/ml.

27. The use according to any one of claims 1 to 2, wherein the therapeutically effective amount is 4.5 mg in a single daily dose, and the single daily dose provides a mean Cₘₐₓ of an active metabolite H4 ranging from 80% to 125% of 14ng/ml.

28. The use according to any one of claims 1 to 2, wherein the therapeutically effective amount is 5.5 mg in a single daily dose, and the single daily dose provides a mean Cₘₐₓ of an active metabolite H4 ranging from 80% to 125% of 17ng/ml.

29. A pharmaceutical composition for use in the treatment and/or prevention of a blood coagulation-related disease, comprising a therapeutically effective amount of compound A and at least one pharmaceutically acceptable excipient, wherein compound A has a particle size D90 of 3µm to 8µm, the excipient comprises a binder, and compound A is (7aS, 2'S)- 2-oxo-clopidogrel represented by:

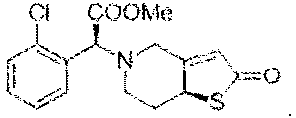

30. The pharmaceutical composition according to claim 29, wherein the therapeutically effective amount is 4 mg to 5.5 mg in a single daily dose, and preferably, the therapeutically effective amount is 4 mg to 5 mg in a single daily dose.

31. The pharmaceutical composition according to claim 29, wherein a single daily dose provides a mean Cₘₐₓ of compound A of 0.8ng/ml to 1.2ng/ml following administration of a therapeutically effective amount of the pharmaceutical composition to a human subject.

32. The pharmaceutical composition according to claim 29, wherein the binder is selected from the group consisting of gum arabic, gelatin, sodium carboxymethylcellulose, methylcellulose, copovidone, polyvidone, hypromellose, and a combination thereof.

33. The pharmaceutical composition according to any one of claims 29 to 31, wherein the pharmaceutical composition further comprises a filler, a disintegrant and a lubricant as a pharmaceutically acceptable excipient.

34. The pharmaceutical composition according to claim 33, wherein the filler is selected from the group consisting of mannitol, sorbitol, calcium sulfate, calcium hydrogen phosphate, calcium carbonate, calcium sulfate dihydrate, sucrose, dextrin, microcrystalline cellulose, lactose, starch, pregelatinized starch, powdered sugar, dextrin, and a combination thereof;
the disintegrant is selected from the group consisting of low-substituted hydroxypropyl cellulose, polacrilin potassium, crospovidone, cross-linked sodium carboxymethyl cellulose, sodium carboxymethyl starch, dry starch, and a combination thereof; and
the lubricant is selected from the group consisting of magnesium stearate, sodium stearic fumarate, silicon dioxide, talc, polyethylene glycol-like substances, hydrogenated vegetable oils, sodium dodecyl sulfate, and a combination thereof.

35. The pharmaceutical composition according to any one of claims 29 to 34, wherein the pharmaceutical composition has a dissolution amount of greater than or equal to 70% within 30 minutes in an acetate buffer solution with a pH of 4.5, as determined according to Method 2 of China Pharmacopoeia 2020 edition General Principles 0931.

36. The pharmaceutical composition according to any one of claims 29 to 34, wherein the pharmaceutical composition has a dissolution percentage of greater than or equal to 85% within 60 minutes in an acetate buffer solution with a pH of 4.5, as determined according to Method 2 of China Pharmacopoeia 2020 edition General Principles 0931.

37. The pharmaceutical composition according to any one of claims 29 to 34, wherein the single daily dose provides a mean Cₘₐₓ/C₁₂ₕ of compound A of 8 to 12.

38. The pharmaceutical composition according to any one of claims 29 to 34, wherein the therapeutically effective amount is 4 mg in a single daily dose, and the single daily dose provides a mean Cₘₐₓ of compound A ranging from 80% to 125% of 0.8 ng/ml.

39. The use according to any one of claims 29 to 34, wherein the therapeutically effective amount is 4.5 mg in a single daily dose, and the single daily dose provides a mean Cₘₐₓ of compound A ranging from 80% to 125% of 0.9 ng/ml.

40. The use according to any one of claims 29 to 34, wherein the therapeutically effective amount is 5.5 mg in a single daily dose, and the single daily dose provides a mean Cₘₐₓ of compound A ranging from 80% to 125% of 1.2 ng/ml.

41. The use according to any one of claims 29 to 34, wherein the therapeutically effective amount is 4 mg in a single daily dose, and the single daily dose provides a mean AUC₍₀₋₄₈ₕ₎ of compound A ranging from 80% to 125% of 2.3h*ng/ml.

42. The use according to any one of claims 29 to 34, wherein the therapeutically effective amount is 4.5 mg in a single daily dose, and the single daily dose provides a mean AUC₍₀₋₄₈ₕ₎ of compound A ranging from 80% to 125% of 3.0h*ng/ml.

43. The use according to any one of claims 29 to 34, wherein the therapeutically effective amount is 5.5 mg in a single daily dose, and the single daily dose provides a mean AUC₍₀₋₄₈ₕ₎ of compound A ranging from 80% to 125% of 3.5h*ng/ml.

44. The use according to any one of claims 29 to 34, wherein the therapeutically effective amount is 4 mg in a single daily dose, and the single daily dose provides a mean AUC₍₀₋ₜ₎ of compound A ranging from 80% to 125% of 2.3h*ng/ml.

45. The use according to any one of claims 29 to 34, wherein the therapeutically effective amount is 4.5 mg in a single daily dose, and the single daily dose provides a mean AUC₍₀₋ₜ₎ of compound A ranging from 80% to 125% of 3.0h*ng/ml.

46. The use according to any one of claims 29 to 34, wherein the therapeutically effective amount is 5.5 mg in a single daily dose, and the single daily dose provides a mean AUC₍₀₋ₜ₎ of compound A ranging from 80% to 125% of 3.5h*ng/ml.

47. The use according to any one of claims 29 to 34, wherein the therapeutically effective amount is 4 mg in a single daily dose, and the single daily dose provides a mean AUC_{(0-∞)} of compound A ranging from 80% to 125% of 2.7h*ng/ml.

48. The use according to any one of claims 29 to 34, wherein the therapeutically effective amount is 4.5 mg in a single daily dose, and the single daily dose provides a mean AUC_{(0-∞)} of compound A ranging from 80% to 125% of 3.5h*ng/ml.

49. The use according to any one of claims 29 to 34, wherein the therapeutically effective amount is 5.5 mg in a single daily dose, and the single daily dose provides a mean AUC_{(0-∞)} of compound A ranging from 80% to 125% of 4.2h*ng/ml.

50. The use according to any one of claims 29 to 34, wherein the therapeutically effective amount is 4 mg in a single daily dose, and the single daily dose provides a mean AUC₍₀₋₁₂ₕ₎ of an active metabolite H4 ranging from 80% to 125% of 13h*ng/ml.

51. The use according to any one of claims 29 to 34, wherein the therapeutically effective amount is 4.5 mg in a single daily dose, and the single daily dose provides a mean AUC₍₀₋₁₂ₕ₎ of an active metabolite H4 ranging from 80% to 125% of 14h*ng/ml.

52. The use according to any one of claims 29 to 34, wherein the therapeutically effective amount is 5.5 mg in a single daily dose, and the single daily dose provides a mean AUC₍₀₋₁₂ₕ₎ of an active metabolite H4 ranging from 80% to 125% of 18.5h*ng/ml.

53. The use according to any one of claims 29 to 34, wherein the therapeutically effective amount is 4 mg in a single daily dose, and the single daily dose provides a mean AUC₍₀₋ₜ₎ of an active metabolite H4 ranging from 80% to 125% of 13h*ng/ml.

54. The use according to any one of claims 29 to 34, wherein the therapeutically effective amount is 4.5 mg in a single daily dose, and the single daily dose provides a mean AUC₍₀₋ₜ₎ of an active metabolite H4 ranging from 80% to 125% of 14h*ng/ml.

55. The use according to any one of claims 29 to 34, wherein the therapeutically effective amount is 5.5 mg in a single daily dose, and the single daily dose provides a mean AUC₍₀₋ₜ₎ of an active metabolite H4 ranging from 80% to 125% of 18.5h*ng/ml.

56. The use according to any one of claims 29 to 34, wherein the therapeutically effective amount is 4 mg in a single daily dose, and the single daily dose provides a mean AUC_{(0-∞)} of an active metabolite H4 ranging from 80% to 125% of 13.5h*ng/ml.

57. The use according to any one of claims 29 to 34, wherein the therapeutically effective amount is 4.5 mg in a single daily dose, and the single daily dose provides a mean AUC_{(0-∞)} of an active metabolite H4 ranging from 80% to 125% of 15h*ng/ml.

58. The use according to any one of claims 29 to 34, wherein the therapeutically effective amount is 5.5 mg in a single daily dose, and the single daily dose provides a mean AUC_{(0-∞)} of an active metabolite H4 ranging from 80% to 125% of 19h*ng/ml.

59. The use according to any one of claims 29 to 34, wherein the therapeutically effective amount is 4 mg in a single daily dose, and the single daily dose provides a mean Cₘₐₓ of an active metabolite H4 ranging from 80% to 125% of 12.5ng/ml.

60. The use according to any one of claims 29 to 34, wherein the therapeutically effective amount is 4.5 mg in a single daily dose, and the single daily dose provides a mean Cₘₐₓ of an active metabolite H4 ranging from 80% to 125% of 14ng/ml.

61. The use according to any one of claims 29 to 34, wherein the therapeutically effective amount is 5.5 mg in a single daily dose, and the single daily dose provides a mean Cₘₐₓ of an active metabolite H4 ranging from 80% to 125% of 17ng/ml.
